# EUROPÄISCHE PATENTSCHRIFT

(11) **EP 1 736 200 B1**
(45) Veröffentlichungstag und Bekanntmachung des Hinweises auf die Patenterteilung: **26.03.2008**
(21) Anmeldenummer: 06012370.0
(22) Anmeldetag: 16.06.2006
(51) Int. Cl.: A61M 39/22, A61M 1/00

(54) **Tastenventil für medizinische Instrumente**
Push-button valve for medical instruments
Soupape à bouton poussoir pour dispositifs médicaux

(30) Priorität: 20.06.2005 DE 102005029756
(43) Veröffentlichungstag der Anmeldung: 27.12.2006
(73) Patentinhaber: Karl Storz GmbH & Co. KG, 78532 Tuttlingen (DE)
(72) Erfinder: Efinger, Andreas, 78604 Rietheim (DE); Riedmüller, Michael, 78144 Tennenbronn (DE)
(74) Vertreter: Weller, Wolfgang

(56) Entgegenhaltungen:
- US-A- 5 348 555
- US-A- 5 554 112
- US-A- 5 658 249

## Beschreibung

Die Erfindung betrifft ein Tastenventil für medizinische Instrumente zum Saugen und Spülen, mit einem Ventilstößel, der über eine vorgespannte Feder dauernd in eine Schließstellung gedrückt wird, und mit einem Betätigungselement, das von einer Hand betätigbar ist, durch das der Ventilstößel gegen die Kraft der Feder in eine Offenstellung bewegbar ist, wobei der Ventilstößel nach Freigabe des Betätigungselements in die Schließstellung bewegt wird, und wobei das Tastenventil lösbar an dem medizinischen Instrument montiert ist.

Die Erfindung betrifft gleichermaßen ein medizinisches Instrument, in dem ein solches Tastenventil eingebaut ist.

Derartige medizinische Instrumente zum Saugen und Spülen, die mit einem solchen Tastenventil ausgestattet sind, werden von der Anmelderin vertrieben und sind in dem Katalog der Anmelderin "HNO-Katalog SI 40", Seite 184 beschrieben.

Medizinische Instrumente zum Saugen und Spülen dienen dazu, bei operativen Eingriffen, insbesondere bei der weitverbreiteten minimal-invasiven Chirurgie, an der Operationsstelle im Körper Spül- oder Absaugvorgänge durchzuführen.

Bei Saug-Spül-Endoskopen wird eine Freispülung des Optik-Objektivs von Blut, Sekret oder Beschlag durchgeführt. Dadurch ist immer ein klarer Blick auf das Operationsfeld gewährleistet.

Derartige Instrumente weisen meist einen Handgriff auf, um vom Operateur während des Eingriffes gehandhabt zu werden. Zur Steuerung des Spül- oder Saugvorganges ist ein Ventil vorgesehen, das vom Operateur betätigt wird.

Dabei haben sich zwei unterschiedliche Ventilausgestaltungen etabliert.

Eine erste Ausgestaltung besteht in einem Druckknopfventil mit Einrastfunktion. Zum Einschalten der Spülung wird der Ventilknopf heruntergedrückt und verbleibt in einer Einrastposition. Zum Ausschalten muss der Ventilknopf nochmals heruntergedrückt werden.

Bei einer zweiten Ausgestaltung, einem so genannten Tastenventil, erfolgt eine Spülung (oder gegebenenfalls Absaugung) nur bei gedrückt gehaltenem Ventilknopf.

Gegenstand der vorliegenden Erfindung ist ein solches Tastenventil.

Im Einsatz können Kontaminationen in das Tastenventil gelangen, so dass es notwendig ist, das Tastenventil nach jedem Einsatz zu reinigen und zu sterilisieren.

Hierzu ist es üblich, das Tastenventil lösbar an dem medizinischen Instrument zum Saugen und Spülen anzubringen, so dass es für den Reinigungsvorgang abgenommen, beispielsweise abgeschraubt, werden kann.

Bei den Tastenventilen besteht nun aufgrund deren Konstruktion das Problem, dass der Ventilstößel immer in seine Schließstellung gedrückt wird. Will man das Tastenventil reinigen, muss man den Ventilstößel betätigen, um diesen in seine Offenstellung zu bringen, so dass das Innere des Ventilkörpers mit der Reinigungsflüssigkeit durchspült werden kann.

Dies ist bei den Tastenventilen äußerst schwierig durchzuführen, da die mit relativ hoher Kraft vorgespannte Feder den Ventilstößel in die Schließstellung drückt.

Ferner ist zu berücksichtigen, dass ein solches Tastenventil die Ausmaße von nur einigen Zentimetern aufweist, so dass es sehr umständlich ist, ein solches Tastenventil in der Hand haltend durch Drücken des Betätigungselements in der Offenstellung zu halten und gleichzeitig zu spülen.

Eine automatisierte Spülung/Reinigung ist konstruktionsbedingt deswegen nicht durchführbar, da ein Durchspülen in der Offenstellung des Ventilstößels nicht möglich ist. Hierzu müsste das Tastenventil wieder in eine Art Halterung, die ein Betätigen des Tastenventils simuliert, eingebaut werden, was konstruktiv sehr aufwendig und äußerst umständlich wäre.

Es ist daher Aufgabe der vorliegenden Erfindung, ein Tastenventil der eingangs genannten Art dahingehend weiterzuentwickeln, dass es einfach gereinigt werden kann.

Erfindungsgemäß wird die Aufgabe dadurch gelöst, dass ein Rastmechanismus vorhanden ist, durch den der Ventilstößel nur in ausgebautem Zustand des Tastenventils in der Offenstellung verrastbar ist, und dass beim Einbauen in das medizinische Instrument der Rastmechanismus automatisch gelöst wird und gelöst bleibt.

Durch diese Ausgestaltung bleibt das Tastenventil, sofern es in dem medizinischen Instrument eingebaut ist, ein Tastenventil, d.h. ein Ventil ohne eine Raststellung. Nur, wenn das Tastenventil ausgebaut wird, ist es möglich, den Ventilstößel in der Offenstellung zu verrasten. Dies kann beispielsweise dadurch erfolgen, dass der Rastmechanismus nur im ausgebauten Zustand betätigt werden kann, nicht jedoch im eingebauten Zustand. Beim Einbauen in das medizinische Instrument wird dieser Rastmechanismus automatisch gelöst und bleibt gelöst, so dass das Tastenventil im eingebauten Zustand immer seine Funktion als Tastenventil behält, also keine Verrastung stattfindet.

Nach Ausbauen des Tastenventils kann der Ventilstößel in die Offenstellung bewegt und dort verrastet werden, so dass für den Reinigungsvorgang der gesamte Innenraum des Ventilkörpers für die Reinigungsflüssigkeit zugänglich ist. Nach gegebenenfalls erfolgtem Sterilisieren wird das gereinigte Tastenventil wieder in das medizinische Instrument eingebaut, und dabei löst sich automatisch der Rastmechanismus und bleibt gelöst, so dass ausgeschlossen ist, dass beim Betätigen des Tastenventils dieses versehentlich in einer Raststellung verbleibt. Somit ist sichergestellt, dass in jeweils eingebautem Zustand das Tastenventil ausschließlich als Tastenventil und nicht als verrastbares Ventil arbeitet.

In einer weiteren Ausgestaltung der Erfindung weist der Rastmechanismus zumindest eine Raste auf, die mit einem Sperrelement in Wirkverbindung steht, durch das die Raste im eingebauten Zustand des Tastenventils vor einem rastenden Eingriff mit dem Ventilstößel gesperrt ist.

Diese Maßnahme hat den Vorteil, dass durch eine mechanisch einfache Maßnahme, nämlich durch das Sperrelement, sichergestellt ist, dass die Raste im eingebauten Zustand vor einem rastenden Eingriff mit dem Ventilstößel gesperrt ist.

In einer weiteren Ausgestaltung der Erfindung ist die Raste des Rastmechanismus bei ausgebautem Tastenventil durch ein einmaliges Betätigen des Betätigungselements in die Raststellung bringbar.

Diese Maßnahme hat den Vorteil, dass die Handhabung für den Reinigungsvorgang sehr vereinfacht ist. Das Tastenventil wird vom medizinischen Instrument abgenommen, und durch einmaliges Drücken des Betätigungselements wird der Rastmechanismus in die Raststellung gebracht, also der Ventilstößel in der Offenstellung verrastet.

Das Tastenventil kann dann in dieser Stellung einem Reinigungs- bzw. Sterilisiervorgang unterworfen werden, der beispielsweise vollautomatisch ablaufen kann.

In einer weiteren Ausgestaltung der Erfindung steht die Raste mit einem Löseelement in Verbindung, durch das die Raste auslösbar ist.

Diese Maßnahme hat den Vorteil, dass ganz gezielt ein Löseelement vorgesehen ist, das die Raste löst, wenn das Tastenventil wieder in das medizinische Instrument eingebaut wird. Dies stellt sicher, dass das Tastenventil auch nach einem Reinigungsvorgang wieder seine Funktion als Tastenventil und nicht als Ventil mit Verrastung im medizinischen Instrument ausübt.

In einer weiteren Ausgestaltung der Erfindung ist das Löseelement derart ausgebildet, dass es beim Einbauen des Tastenventils in das medizinische Instrument die Raste löst und dauernd in der gelösten Stellung belässt.

Diese Maßnahme hat den Vorteil, dass nicht nur das Auslösen der Raste durch das Löseelement bewirkt wird, sondern dass dieses auch dafür sorgt, dass die Raste ständig in der gelösten Stellung verbleibt, solange das Tastenventil in das medizinische Instrument eingebaut ist.

In einer weiteren Ausgestaltung der Erfindung sind Sperrelement und Löseelement durch ein Bauteil gebildet.

Diese Maßnahme hat den Vorteil, dass durch möglichst wenig Bauelemente der zusätzliche Rastmechanismus bewerkstelligt werden kann.

In einer weiteren Ausgestaltung der Erfindung ist das Sperrelement durch Federkraft in Richtung einer nicht sperrenden Position beaufschlagbar.

Diese Maßnahme hat den Vorteil, dass bei ausgebautem Tastenventil das Sperrelement durch die Feder automatisch in eine nichtsperrende Stellung bewegt wird, so dass sichergestellt ist, dass nunmehr der Rastmechanismus in diesem Zustand wirken kann. Dies eröffnet die Möglichkeit, das Sperrelement so auszugestalten, dass beim Einbauen des Tastenventils das Sperrelement gegen die Federkraft in die entgegengesetzte Richtung, also in die sperrende Richtung, verschoben wird. Der zuvor erwähnte Automatismus ist durch sehr einfache mechanische Mittel gewährleistet.

In einer weiteren Ausgestaltung der Erfindung ist die Raste als elastisch federndes Element ausgestaltet, das mit einer Eingriffsstelle am Ventilstößel in sperrenden Eingriff kommt, sofern die Raste vom Sperrelement freigegeben ist und der Ventilstößel in die Offenstellung bewegt wird.

Diese Maßnahme hat den Vorteil, dass die Verrastung ohne zusätzliche Handhabung der Raste erfolgen kann. Ist die Raste nach dem Ausbauen des Tastenventils aus dem medizinischen Instrument freigegeben, so ist die Raste quasi schon aktiv, und es braucht nur der Ventilstößel in die Offenstellung bewegt zu werden, um die federbelastete Raste in sperrenden Eingriff mit dem Ventilstößel zu bringen. Die Raste verbleibt aufgrund der Federkraft während des gesamten Reinigungsvorgangs in dieser Stellung.

In einer weiteren Ausgestaltung der Erfindung ist das Sperrelement als sich axial erstreckender federbelasteter Stift ausgebildet.

Diese Maßnahme hat den Vorteil, dass ein sich axial erstreckendes Sperrelement kein von dem Ventil sperrig abstehendes Bauelement darstellt. Ferner kann die axiale Bewegung beim Einsetzen bzw. Ausbauen des Tastenventils gleich zur Steuerung des Sperrelements herangezogen werden, dahingehend, dass dabei das Sperrelement entsprechend axial verschoben wird.

In einer weiteren Ausgestaltung der Erfindung ist die Raste als radial federndes Element ausgebildet.

Diese Maßnahme hat den Vorteil, dass die Raste wenig raumergreifend in den Ventilkörper eingebaut werden kann. Durch den radialen Bewegungsweg kann bereits durch eine kleine radiale Verschiebung der notwendig mechanisch feste Eingriff geschaffen werden, um den Ventilstößel in seiner verrasteten Stellung zu halten, insbesondere auch dauernd zu halten während des gesamten Reinigungsvorgangs.

In einer weiteren Ausgestaltung der Erfindung ist der Stift an dem dem federnden Element zugewandten Ende mit einer Schräge versehen, die mit dem radial federnden Element beim Lösen der Raste in Eingriff kommt.

Diese Ausgestaltung ermöglicht, durch einfache und kompakte Bauelemente die axiale Bewegung des Stiftes über die Schräge in eine radiale Ausrückbewegung des radial federnden Elementes umzusetzen. Hierdurch ist ein sanftes, harmonisches Steuern des Lösens des Rastmechanismus möglich.

Wie bereits erwähnt, werden üblicherweise die Tastenventile über eine axiale Vorschubbewegung in das medizinische Element eingebaut, so dass nach und nach durch axiales Verschieben des Stiftes des Sperrelementes das radial bewegliche, federnde Rastelement aus der Raststellung mittels der vorgeschobenen Schräge herausbewegt wird.

In einer weiteren Ausgestaltung der Erfindung ist der Stift als an der Außenseite des Ventilkörpers angeordneter Stift ausgebildet.

Diese Maßnahme hat den konstruktiven Vorteil, dass keine grundsätzlichen Änderungen des Ventilkörpers in seinen Maßen notwendig sind, sondern dass der Stift an seiner Außenseite angebracht werden kann und dieser dann beim Einsetzen des Tastenventils an einen entsprechenden Anschlag am medizinischen Instrument trifft, wodurch der Stift dann längs der Außenseite des Ventilkörpers bewegt wird.

In einer weiteren Ausgestaltung der Erfindung ist das radial federnde Element als radial nach innen vorgespannte Stabfeder ausgebildet.

Diese Maßnahme hat den Vorteil, dass die Raste als ein sehr kleines, in den Ventilkörper einfach integrierbares Bauelement ausgebildet ist.

In einer weiteren Ausgestaltung der Erfindung ist im Ventilstößel eine Nut ausgespart, in die die radial vorgespannte Stabfeder in sperrenden Eingriff eindrückbar ist.

Diese Maßnahme hat den Vorteil, dass, wie zuvor erwähnt, durch diesen Eingriff eine auf Dauer feste Verriegelung des Ventilstößels in seiner Offenstellung sichergestellt ist. Somit kann das ausgebaute Tastenventil auch mechanischen Belastungen beim Reinigen, Spülen, Waschen und Transportieren ausgesetzt werden, ohne dass die Gefahr besteht, dass der Rastmechanismus gelöst wird, da die radial in die Nut eingerastete federbelastete Stiftfeder auch bei mechanischen Schocks nicht aus der Nut austritt.

In einer weiteren Ausgestaltung der Erfindung ist der Ventilstößel in einer Ventilanschraubung aufgenommen, die zumindest zwei Fingermulden aufweist.

Diese Maßnahme hat den Vorteil, dass über die Fingermulden das Tastenventil per Hand, also auch ohne ein Werkzeug, vom medizinischen Instrument abgedreht oder abgenommen und nach der Reinigung wieder eingesetzt werden kann.

Auch diese Maßnahme trägt zusätzlich zur Vereinfachung des Reinigungsvorganges bei. Diese Ausgestaltung der Fingermulden wird zusätzlich als eigenständige Erfindung angesehen.

In einer weiteren Ausgestaltung der Erfindung ist eine Einschnittkontur der Fingermulden derart ausgestaltet, dass eine Muldenflanke in Richtung des Herausdrehens stark ausgeprägt ist.

Diese Maßnahme hat den Vorteil, dass durch diese starke Ausprägung der Muldenflanke beim Herausdrehen eine relativ große Angriffsfläche für die Fingerkuppe zur Verfügung steht, über die ein ausreichend großer tangentialer Druck ausgeübt werden kann, um das Tastenventil herauszudrehen. Dadurch kann bei diesem relativ kleinen Bauteil über die stark ausgeprägte Flanke der Mulde jeweils über die Finger ein ausreichender Druck ausgeübt werden, um das Tastenventil ohne Zuhilfenahme irgendwelcher Werkzeuge aus dem medizinischen Instrument auszubauen.

In einer weiteren Ausgestaltung der Erfindung ist die Muldenflanke in Richtung des Herausdrehens in radialer Richtung weiter ansteigend als die gegenüberliegende Muldenfläche in Richtung des Eindrehens.

Diese Maßnahme hat den Vorteil, dass die zuvor erwähnte größere Kraft- oder Druckausübung in Richtung des Herausdrehens durch den Finger möglich ist, in entgegengesetzter Richtung aber beim Eindrehen ein solch hoher Druck nicht ausgeübt werden kann, so dass das Tastenventil beim Einsetzen oder Eindrehen nicht zu fest sitzt oder gar überdreht werden kann.

In der konkreten Ausgestaltung erstreckt sich die stärker ausgeprägte Muldenflanke, von der Muldensenke aus gesehen, steiler sowie radial länger als die weniger ausgeprägte Muldenflanke.

In einer weiteren Ausgestaltung der Erfindung geht die Muldenflanke in Richtung des Eindrehens in eine umfängliche Abrundung über.

Diese Maßnahme hat den Vorteil, dass beim Eindrehen des Tastenventils nach Erreichen einer gewünschten Endposition der Finger beim Versuch, das Tastenventil weiterzudrehen, sich über die umfängliche Abrundung bewegt und dabei aus der Mulde herausgleitet, so dass keine weiteren unnötigen Kräfte ausgeübt werden können.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachstehend anhand eines ausgewählten Ausführungsbeispiels im Zusammenhang mit den beiliegenden Zeichnungen näher beschrieben und erläutert. Es zeigen:
- Fig. 1: eine Seitenansicht eines medizinischen Instruments zum Saugen und Spülen, in dessen Griff ein erfindungsgemäßes Tastenventil eingebaut ist,
- Fig. 2: stark schematisiert eine Seitenansicht des im Griff eingebauten Tastenventils,
- Fig. 3: einen Längsschnitt des Tastenventils von Fig. 2, allerdings um 180° um dessen Längsachse verdreht,
- Fig. 4: eine der Fig. 2 entsprechende Darstellung des Tastenventils, allerdings in ausgebautem Zustand des Tastenventils,
- Fig. 5: eine dem Schnitt von Fig. 3 vergleichbare Darstellung, aber des ausgebauten Tastenventils,
- Fig. 6: eine teilweise aufgebrochene Draufsicht auf das Ventil von Fig. 3 im eingebauten entriegelten Zustand,
- Fig. 7: eine entsprechende Darstellung des Ventils von Fig. 5 in ausgebautem verriegelten Zustand,
- Fig. 8: eine der Schnittdarstellung von Fig. 5 vergleichbare Darstellung des ausgebauten Tastenventils nach dem einmaligen Drücken des Betätigungselements und der Verrastung für den Reinigungsvorgang, und
- Fig. 9: eine der Darstellung von Fig. 7 vergleichbare Darstellung im verrasteten Zustand.

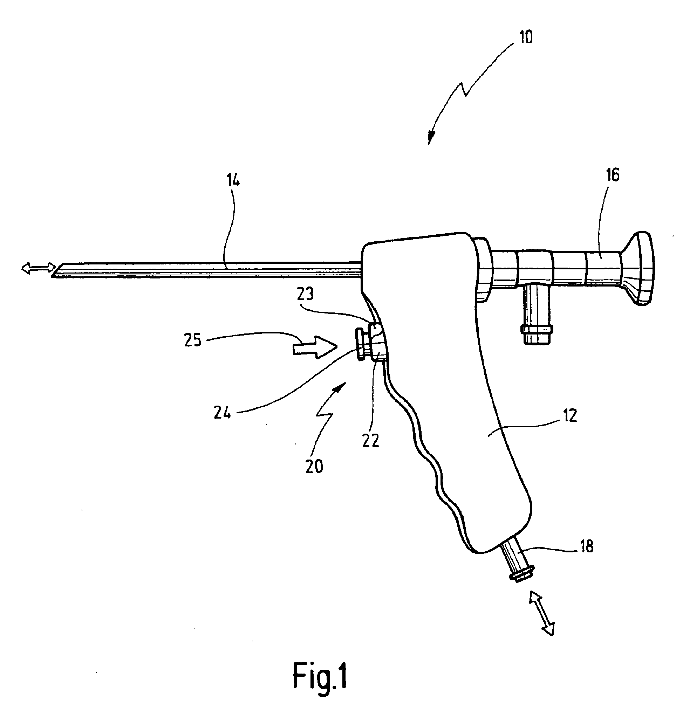

Ein in Fig. 1 dargestelltes medizinisches Instrument zum Saugen und Spülen ist in seiner Gesamtheit mit der Bezugsziffer 10 bezeichnet.

Das medizinische Instrument 10 weist einen pistolenartigen Griff 12 auf, der einen langerstreckten hohlen Saug- und Spülschaft 14 aufweist. In den Schaft 14 kann zusätzlich eine Optik 16 eingesetzt sein oder eingeschoben werden, um beispielsweise bei minimal-invasiven Operationstechniken eine Operationsstelle visuell beobachten zu können. Am äußeren Ende des Griffes 12 ist ein Anschluss 18 zum Anschluss an eine Vorrichtung zum Saugen und Spülen vorgesehen. Über den Anschluss 18 kann beim Spülen eine Spülflüssigkeit zugeführt werden, die durch hier nicht dargestellte Leitungen im Griff 12 zum Schaft 14 und dort an dessen distales Ende geführt wird, wo die Spülflüssigkeit austritt. Die Spülflüssigkeit kann beispielsweise dazu dienen, das distale Ende der Optik 16 freizuspülen, oder kann auch dazu dienen, den Operationsort freizuspülen. Es kann auch über den Schaft 14 und den Anschluss 18 die Spülflüssigkeit wieder abgesaugt werden.

Im Griff 12 ist distalseitig ein Tastenventil 20 eingesetzt. Das Tastenventil 20 ist über eine kragenartige Anschraubung 22 mit dem Griff 12 verbunden. Es sind zwei diametral gegenüberliegende Fingermulden 23 vorgesehen, über die die Anschraubung 22 mit zwei Fingern erfasst werden kann, um beispielsweise das Tastenventil 20 vom Griff 12 abzuschrauben.

Das Tastenventil 20 sitzt, wie an sich bekannt ist, im Strömungspfad zwischen Anschluss 18 und Schaft 14.

Das Tastenventil 20 weist ein Betätigungselement 24 in Form einer Taste auf, die gegen die Kraft einer Feder eingedrückt werden kann, wie das nachfolgend noch erläutert wird. Wird das Betätigungselement 24 losgelassen, sperrt das Tastenventil 20 den Fluss.

Wird das Betätigungselement 24 beispielsweise durch einen Finger des Operateurs, der das medizinische Instrument 10 in der Hand hält, betätigt, wie durch einen Pfeil 25 angedeutet ist, öffnet das Tastenventil 20, und ein Fluss, beispielsweise das Durchfließen einer Spülflüssigkeit, wird ermöglicht.

Wird das Betätigungselement 24 freigegeben, kehrt es aufgrund der Beaufschlagung mit der Federkraft automatisch wieder in die sperrende Stellung zurück.

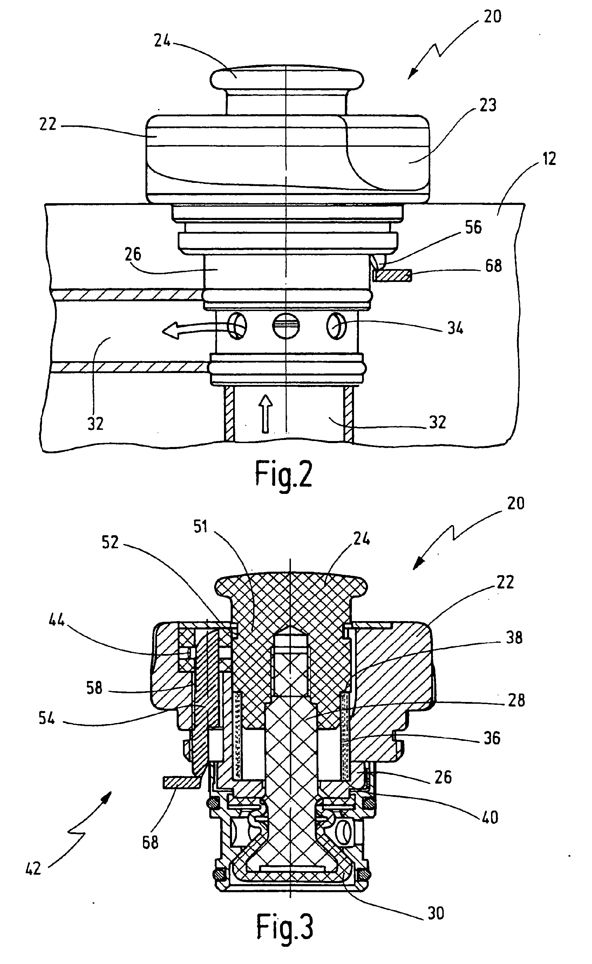

In Fig. 2 ist das Tastenventil 20 in seiner Außenkontur dargestellt, wobei der Einbau in die Leitung 32 im Inneren des Griffes 12 nur sehr schematisch dargestellt ist.

Aus Fig. 2 ist zu erkennen, dass die Anschraubung 22 über die Fingermulden 23 durch zwei Finger ergriffen werden kann, um das Tastenventil 20 vom medizinischen Instrument 10 abzudrehen oder in dieses einzusetzen.

Aus der Längsschnittdarstellung von Fig. 3 ist zu erkennen, dass im Gehäuse 26 ein Ventilstößel 28 aufgenommen ist. Der Ventilstößel 28 ist mit dem Betätigungselement 24 verschraubt und bildet eine einzige Baueinheit. Am unteren Ende trägt der Ventilstößel 28 eine Dichtung 30, die dafür sorgt, dass ein Fluss durch das Tastenventil 20 in der Richtung der Strömungspfeile in Fig. 2 gesperrt ist. Der Zusammenbau aus Betätigungselement 24 und Ventilstößel 28, nachfolgend nur noch Ventilstößel 28 genannt, wird durch die Kraft einer Feder 36 derart vorgespannt, dass der Ventilstößel 28 in die in Fig. 3 dargestellte Schließstellung gedrückt wird. Die Feder 36 ist als Schaubenlinienfeder ausgestaltet, die den Körper des Ventilstößels 28 umrundet und sich einerseits auf einer Schulter 38 am Ventilstößel 28 und andererseits an einem Absatz 40 des Gehäuses 26 abstützt.

Zum Öffnen des Tastenventils 20 wird, wie vorstehend im Zusammenhang mit Fig. 1 beschrieben, mit dem Finger auf das Betätigungselement 24 gedrückt und der Ventilstößel 28 gegen die Kraft der Feder 36 in die Offenstellung bewegt, so dass dann ein Fluss von Spülmedium oder Saugmedium durch die Leitung 32 erfolgen kann. Wird das Betätigungselement 24 wieder freigegeben, kehrt der Ventilstößel 28 in die Schließstellung zurück.

In Fig. 3 ist der erfindungsgemäße Rastmechanismus in seiner Gesamtheit mit der Bezugsziffer 42 versehen.

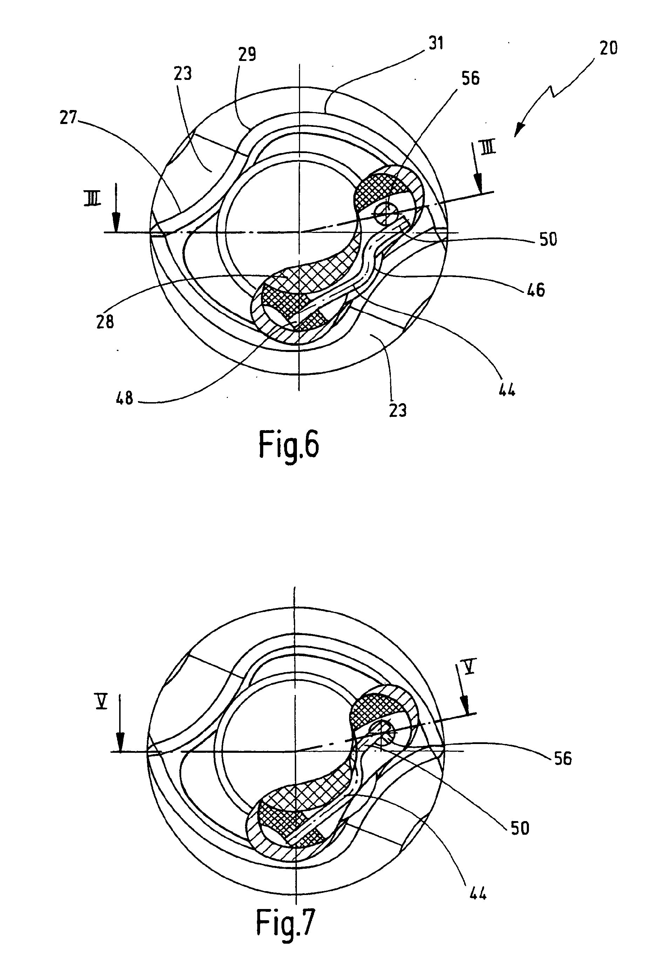

Der Rastmechanismus 42 weist eine Raste 44 auf, die in einem Ringraum um den Ventilstößel 28 herum angeordnet ist, wie das insbesondere aus der Schnittdarstellung von Fig. 6 ersichtlich ist.

Die Raste 44 ist als gekröpfte Stabfeder 46 ausgebildet, deren festes Ende 48 im Gehäuse 26 fest verankert ist, während deren gegenüberliegendes freies Ende 50 frei beweglich ist, nämlich radial nach innen und außen.

Im Körper des Ventilstößels 28, hier im Körper 51 des Betätigungselements 24, ist eine umfängliche äußere Ringnut 52 eingeschnitten, die so ausgestaltet ist, dass in diese das freie bewegliche Ende 50 der Raste 44 eintreten kann, wenn die Ringnut 52 auf Höhe des freien Endes 50 der gekröpften Stabfeder 46 zum Liegen kommt.

In Fig. 3 und 6 ist ersichtlich, dass das Einrasten der Raste 44 in die Ringnut 52 durch ein Sperrelement 54 gesperrt ist.

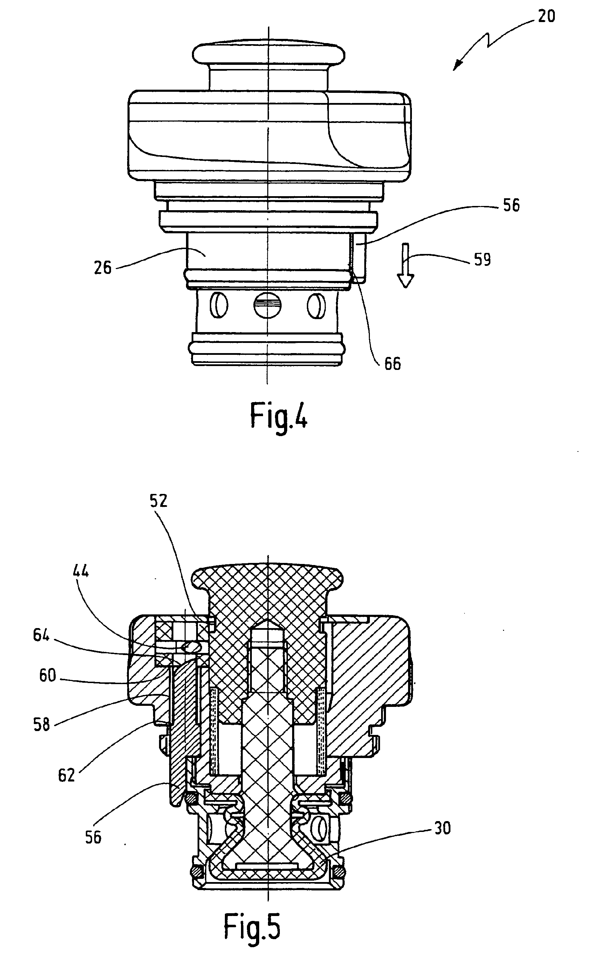

Das Sperrelement 54 ist als ein Stift 56 ausgebildet, der in einer seitlichen axialen Bohrung im Körper der Anschraubung 22 aufgenommen ist. Der Stift 56 wird durch die Kraft einer Feder 58 beaufschlagt, die sich zwischen einem Widerlager 60 (siehe insbesondere Fig. 5) im Inneren der Anschraubung 22 und einem Absatz 62 am Stift 56 erstreckt.

Die Feder 58 ist so vorgespannt, dass sie dazu neigt, den Stift 56 (in den Darstellungen) axial nach unten zu verschieben.

Ist das Tastenventil 20 in das medizinische Instrument 10 eingebaut, wie das in den Figuren 1 bis 3 dargestellt ist, ist allerdings diese Bewegung des Stiftes 56 dadurch gehindert, dass der Stift 56 auf einem Anschlag 68 im Inneren des medizinischen Instrumentes ruht.

Wie insbesondere aus Fig. 3 ersichtlich ist, ist also in diesem eingebauten Zustand des Tastenventils 20 der Stift 56 so weit axial nach oben verschoben, dass er sich zwischen dem freien Ende 50 der gekröpften Stabfeder 46 und dem Körper 51 des Ventilstößels 28 befindet. Somit ist in diesem eingebauten Zustand das freie Ende 50 dauernd davor gesperrt, dass die gekröpfte Stabfeder 46 der Raste 44 in die Ringnut 52 eintreten kann.

Somit arbeitet das Tastenventil 20 in diesem Zustand als Tastenventil ohne eine Verrastung.

Wird das Tastenventil 20 nunmehr aus dem medizinischen Instrument 10 ausgebaut, wobei dieser Zustand in den Figuren 4 und 5 dargestellt ist, wird der Stift 56 durch die vorgespannte Feder 58 so weit nach unten bewegt, bis das freie Ende 50 der gekröpften Stabfeder 46 radial frei beweglich ist.

Aus der Schnittdarstellung von Fig. 6 ist ersichtlich, dass jede Fingermulde 23 eine etwas asymmetrische Kontur aufweist. Eine Muldenflanke 27 ist stärker ausgeprägt als die gegenüberliegende Muldenflanke 29. Die Muldenflanke 27 dient als Angriffsfläche für einen Finger beim Herausdrehen des Tastenventils aus dem medizinischen Instrument 10, wobei diese Drehbewegung entgegen dem Uhrzeigersinn verläuft. Aus Fig. 6 ist zu erkennen, dass die Muldenflanke 27 stärker und auch radial weiter ansteigt als die gegenüberliegende Muldenflanke 29. Ferner ist zu erkennen, dass die Muldenflanke 29 in eine umfängliche Abrundung 31 übergeht, die sanft bis zur äußeren Umfangskante umläuft.

Beim Eindrehen des Tastenventils 20, was im Uhrzeigersinn erfolgt, kann zwar über die Muldenflanke 29 die ausreichende Kraft ausgeübt werden, um das Tastenventil 20 einzudrehen, auch fest sitzend einzudrehen, ein Überdrehen ist aber nicht möglich. Wird nämlich ein zu großer Kraftaufwand durch die Finger ausgeübt, rutscht der Finger über die sanfte Abrundung 31 aus der Fingermulde 23 heraus, und ein weiteres Drehen ist nicht möglich.

Wird nach dem Ausbauen des Tastenventils 20 das Betätigungselement 24 und somit auch der Ventilstößel 28 einmal betätigt, kommt die Ringnut 52 vor dem freien Ende 50 der Raste 44 zum Liegen, so dass diese in einer radial nach innen gerichteten Einrastbewegung in die Ringnut 52 einrasten kann.

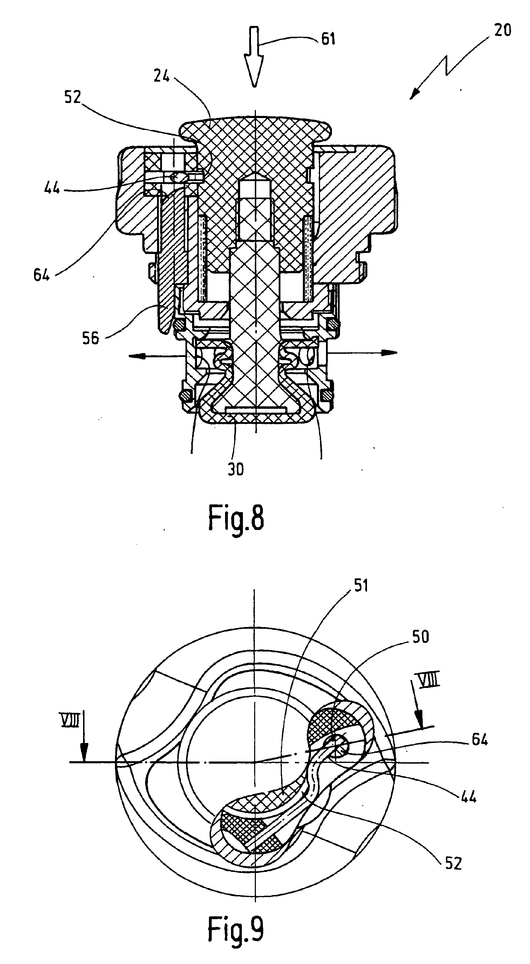

Diese Situation ist in den Figuren 8 und 9 dargestellt.

Wie durch den Pfeil 61 dargestellt, wurde das Betätigungselement 24 eingedrückt, und die Raste 44 ist in die Ringnut 52 eingerastet und hält nunmehr den Ventilstößel 28 in seiner Offenstellung. Die Dichtung 30 ist dabei von deren Sitz abgehoben. Der gesamte, mit den kontaminierten Flüssigkeiten in Berührung tretende innere Bereich des Tastenventils 20 ist nunmehr in dieser Stellung für Reinigungsflüssigkeiten zugänglich, wie das durch die Strömungspfeile angedeutet ist. Es ist auch ersichtlich, dass durch diesen Eingriff der Ventilstößel 28 dauernd in seiner Offenstellung gehalten wird.

Beim Einsetzen des Tastenventils in das medizinische Instrument 10 nach dem Reinigen trifft das in der Darstellung in Fig. 8 untere Ende des Stiftes 56 auf den entsprechenden Anschlag 68 (siehe Figuren 2 und 3), und beim weiteren Eintreiben oder Einsetzen wird dieser Stift 56 nach oben in Richtung der Raste 44 verschoben. An diesem der Raste 44 zugewandten Ende ist der Stift 56 mit einer Schräge 64 versehen.

Die Schräge 64 ist nun so ausgestaltet, dass sie mit dem freien Ende 50 in Eingriff kommt und dabei dieses freie Ende 50 radial nach außen verschiebt, wobei die Raste 44 aus dem rastenden Eingriff mit der Nut 52 herausbewegt wird.

Dieser Übergang ist beispielsweise von Fig. 7 zu Fig. 6 deutlich ersichtlich, bzw. beim Übergang von Fig. 8 zu Fig. 3.

Da, wie zuvor beschrieben, im eingebauten Zustand der Stift 56 des Tastenventils 20 auf dem Anschlag 68 ruht, ist ein Bewegen des Stiftes 56 in Richtung der Beaufschlagung durch die Feder 58 nicht mehr möglich, so dass das Sperrelement 54 dauernd in seiner sperrenden Stellung verbleibt, wie in Fig. 3 dargestellt. Dadurch ist sichergestellt, dass im eingebauten Zustand das Tastenventil 20 ausschließlich als Tastenventil ohne Sperrverriegelung arbeitet.

Aus der Darstellung von Fig. 2 und Fig. 4 ist zu entnehmen, dass der Stift 56 an der Außenseite 66 des Gehäuses 26 angeordnet ist und dessen freies unteres Ende entsprechend beim Einsetzen auf dem Anschlag 68 zum Liegen kommt.

## Patentansprüche

1. Tastenventil für medizinische Instrumente (10) zum Saugen und Spülen, mit einem Ventilstößel (28), der über eine vorgespannte Feder (36) dauernd in eine Schließstellung gedrückt wird, und mit einem Betätigungselement (24), das von einer Hand betätigbar ist, durch das der Ventilstößel (28) gegen die Kraft der Feder (36) in eine Offenstellung bewegbar ist, wobei der Ventilstößel (28) nach Freigabe des Betätigungselements (24) in die Schließstellung bewegt wird, und wobei das Tastenventil (20) lösbar am medizinischen Instrument (10) montierbar ist, **dadurch gekennzeichnet, dass** ein Rastmechanismus (42) vorgesehen ist, durch den der Ventilstößel (28) nur im ausgebauten Zustand des Tastenventils (20) in der Offenstellung verrastbar ist, und dass beim Einbauen in das medizinische Instrument (10) der Rastmechanismus (42) automatisch gelöst wird und gelöst bleibt.

2. Tastenventil nach Anspruch 1, **dadurch gekennzeichnet, dass** der Rastmechanismus (42) zumindest eine Raste (44) aufweist, die mit einem Sperrelement (54) in Wirkverbindung steht, durch das die Raste (44) in eingebautem Zustand des Tastenventils (20) vor einem rastenden Eingriff mit dem Ventilstößel (28) gesperrt ist.

3. Tastenventil nach Anspruch 2, **dadurch gekennzeichnet, dass** die Raste (44) des Rastmechanismus (42) bei ausgebautem Tastenventil (20) durch einmaliges Betätigen des Betätigungselements (24) in die Raststellung bringbar ist.

4. Tastenventil nach Anspruch 2 oder 3, **dadurch gekennzeichnet, dass** die Raste (44) mit einem Löseelement in Wirkverbindung steht, durch das die Raste (44) lösbar ist.

5. Tastenventil nach Anspruch 4, **dadurch gekennzeichnet, dass** das Löseelement derart ausgebildet ist, dass es beim Einbauen des Tastenventils (20) in das medizinische Instrument (10) die Raste (44) löst und dauernd in der gelösten Stellung belässt.

6. Tastenventil nach Anspruch 4 oder 5, **dadurch gekennzeichnet, dass** Sperrelement (54) und Löseelement durch ein Bauteil gebildet sind.

7. Tastenventil nach einem der Ansprüche 2 bis 6, **dadurch gekennzeichnet, dass** das Sperrelement (54) durch Federkraft (58) in Richtung einer nicht sperrenden Position beaufschlagt ist.

8. Tastenventil nach einem der Ansprüche 2 bis 7, **dadurch gekennzeichnet, dass** die Raste (44) als elastisch federndes Element (46) ausgestaltet ist, das mit einer Eingriffstelle am Ventilstößel (28) in sperrenden Eingriff kommt, sofern die Raste (44) vom Sperrelement (54) freigegeben ist und der Ventilstößel (28) in die Offenstellung bewegt wird.

9. Tastenventil nach einem der Ansprüche 2 bis 8, **dadurch gekennzeichnet, dass** das Sperrelement (54) als sich axial erstreckender, federbelasteter Stift (56) ausgebildet ist.

10. Tastenventil nach Anspruch 8 oder 9, **dadurch gekennzeichnet, dass** die Raste (44) als radial federndes Element (46) ausgebildet ist.

11. Tastenventil nach Anspruch 9 oder 10, **dadurch gekennzeichnet, dass** der Stift (56) an dem dem federnden Element (46) zugewandten Ende eine Schräge (64) aufweist, die mit dem radial federnden Element (46) beim Lösen der Raste (44) in Eingriff kommt.

12. Tastenventil nach einem der Ansprüche 9 bis 11, **dadurch gekennzeichnet, dass** der Stift (56) als an der Außenseite (66) des Ventilkörpers angeordneter Stift (56) ausgebildet ist.

13. Tastenventil nach einem der Ansprüche 8 bis 12, **dadurch gekennzeichnet, dass** das federnde Element (46) als radial nach innen vorgespannte Stabfeder (46) ausgebildet ist.

14. Tastenventil nach Anspruch 13, **dadurch gekennzeichnet, dass** im Ventilstößel (28) eine Nut (52) ausgespart ist, in die die radial vorgespannte Stabfeder (46) in sperrenden Eingriff einrückbar ist.

15. Tastenventil nach einem der Ansprüche 1 bis 14, **dadurch gekennzeichnet, dass** der Ventilstößel (28) in einer Ventilanschraubung (22) aufgenommen ist, die zumindest zwei Fingermulden (23) aufweist.

16. Tastenventil nach Anspruch 15, **dadurch gekennzeichnet, dass** eine Einschnittkontur der Fingermulden (23) derart ausgebildet ist, dass eine Muldenflanke (27) in Richtung des Herausdrehens stark ausgeprägt ist.

17. Tastenventil nach Anspruch 16, **dadurch gekennzeichnet, dass** die Muldenflanke (27) in Richtung des Herausdrehens in radialer Richtung weiter ansteigt als die gegenüberliegende Muldenflanke (29) in Richtung des Eindrehens.

18. Tastenventil nach Anspruch 17, **dadurch gekennzeichnet, dass** die Muldenflanke (29) in Richtung des Eindrehens in eine umfängliche Abrundung (31) übergeht.

19. Medizinisches Instrument zum Saugen und Spülen, **dadurch gekennzeichnet, dass** es ein Tastenventil (20) nach einem der Ansprüche 1 bis 18 eingebaut aufweist.

## Claims

1. Press-down valve for medical instruments (10) for suction and irrigation, with a valve stem (28) which is pressed permanently into a closure position via a pretensioned spring (36), and with an actuation element (24) which can be activated by one hand and by means of which the valve stem (28) can be moved counter to the force of the spring (36) into an open position, and, after release of the actuation element (24), the valve stem (28) is moved into the closure position, and the press-down valve (20) can be mounted on the medical instrument (10) in a releasable manner, **characterized in that** a catch mechanism (42) is provided by means of which the valve stem (28) can be locked in the open position only when the press-down valve (20) has been removed from the medical instrument (10), and, when it is installed in the medical instrument (10), the catch mechanism (42) is automatically released and remains released.

2. Press-down valve of claim 1, **characterized in that** the catch mechanism (42) comprises at least one catch (44) operatively connected to a blocking element (54) by means of which the catch (44), in the installed state of the press-down valve (20), is blocked against locking engagement with the valve stem (28).

3. Press-down valve of claim 2, **characterized in that** the catch (44) of the catch mechanism (42), in the removed state of the press-down valve (20), can be brought into the catch position by a single activation of the actuation element (24).

4. Press-down valve of claims 2 or 3, **characterized in that** the catch (44) is operatively connected to a release element by means of which the catch (44) can be released.

5. Press-down valve of claim 4, **characterized in that** the release element is designed in such a way that, upon installation of the press-down valve (20) into the medical instrument (10), it releases the catch (44) and leaves it permanently in the released position.

6. Press-down valve of claims 4 or 5, **characterized in that** the blocking element (54) and release element are formed by one component part.

7. Press-down valve of anyone of claims 2 through 6, **characterized in that** the blocking element (54) is acted on by a spring force (58) in the direction of a non-blocking position.

8. Press-down valve of anyone of claims 2 through 7, **characterized in that** the catch (44) is designed as an elastically resilient element (46) which comes into blocking engagement with an engagement site on the valve stem (28), as long as the catch (44) is freed from the blocking element (54) and the valve stem (28) is moved into the open position.

9. Press-down valve of anyone of claims 2 through 8, **characterized in that** the blocking element (54) is designed as an axially extending, spring-loaded pin (56).

10. Press-down valve of claims 8 or 9, **characterized in that** the catch (44) is designed as a radially resilient element (46).

11. Press-down valve of claims 9 or 10, **characterized in that** the pin (56) has, at the end directed toward the resilient element (46), a bevel (64) which comes into engagement with the radially resilient element (46) upon release of the catch (44).

12. Press-down valve of anyone of claims 9 through 11, **characterized in that** the pin (56) is designed as a pin (56) arranged on the outer face (66) of the valve body.

13. Press-down valve of anyone of claims 8 through 12, **characterized in that** the resilient element (46) is designed as a radially inwardly pretensioned bar spring (46).

14. Press-down valve of claim 13, **characterized in that** a groove (52) is formed in the valve stem (28), the radially pretensioned bar spring (46) being able to move into blocking engagement in the groove (52).

15. Press-down valve of anyone of claims 1 through 14, **characterized in that** the valve stem (28) is received in a valve screw-on piece (22) which has at least two finger molds (23).

16. Press-down valve of claim 15, **characterized in that** an indented contour of the finger molds (23) is designed in such a way that one mold flank (27) is strongly pronounced in the direction of turning out.

17. Press-down valve of claim 16, **characterized in that** the mold flank (27) rises further in the radial direction in the direction of turning out than the opposite mold flank (29) does in the direction of turning in.

18. Press-down valve of claim 17, **characterized in that** the mold flank (29) merges into a peripheral rounding (31) in the direction of turning in.

19. A medical instrument for suction and irrigation, **characterized in that** the instrument comprises a press-down valve (20) as claimed in anyone of claims 1 through 18.

## Revendications

1. Vanne à touches pour instruments (10) médicaux pour l'aspiration et de lavage, comprenant un poussoir de soupape (28), qui est appuyé au moyen d'un ressort (36) prétendu de façon permanente dans une position de fermeture, et un élément d'actionnement (24), qui peut être actionné par une main, par lequel le poussoir de soupape (28) peut être déplacé contre la force du ressort (36) dans une position ouverte, le poussoir de soupape (28) étant déplacé après la libération de l'élément d'actionnement (24) dans la position de fermeture, et la soupape à touches (20) pouvant être montée de façon amovible sur l'instrument (10) médical, **caractérisée en ce qu'**un mécanisme à cran (42) est prévu, par lequel le poussoir de soupape (28) ne peut être encliqueté dans la position ouverte que lorsque la soupape à touches (20) est enlevée et **en ce que**, lors du montage sur l'instrument (10) médical, le mécanisme à cran (42) est desserré automatiquement et reste desserré.

2. Soupape à touches selon la revendication 1, **caractérisée en ce que** le mécanisme à cran (42) présente au moins un cran (44), qui est en liaison active avec un élément de blocage (54) par lequel le cran (44) est bloqué, lorsque la soupape à touches (20) est montée, avant un engagement par encliquetage avec le poussoir de soupape (28).

3. Soupape à touches selon la revendication 2, **caractérisée en ce que** le cran (44) du mécanisme à cran (42) peut être amené dans la position d'encliquetage, lorsque la soupape à touches (20) est enlevée, par un unique actionnement de l'élément d'actionnement (24).

4. Soupape à touches selon la revendication 2 ou 3, **caractérisée en ce que** le cran (44) est en liaison active avec un élément de desserrage, par lequel le cran (44) peut être desserré.

5. Soupape à touches selon la revendication 4, **caractérisée en ce que** l'élément de desserrage est réalisé de telle sorte que, lorsque la soupape à touches (20) est montée dans l'instrument (10) médical, il détache le cran (44) et le laisse de façon permanente dans la position détachée.

6. Soupape à touches selon la revendication 4 ou 5, **caractérisée en ce que** l'élément de blocage (54) et l'élément de desserrage sont formés par un composant.

7. Soupape à touches selon l'une quelconque des revendications 2 à 6, **caractérisée en ce que** l'élément de blocage (54) est sollicité par une force de ressort (58) en direction d'une position non bloquante.

8. Soupape à touches selon l'une quelconque des revendications 2 à 7, **caractérisée en ce que** le cran (44) est conçu comme un élément (46) à élasticité de ressort, qui vient en prise bloquante avec un point d'engagement sur le poussoir de soupape (28), dans la mesure où le cran (44) est libéré de l'élément de blocage (54) et le poussoir de soupape (28) est déplacé dans la position ouverte.

9. Soupape à touches selon l'une quelconque des revendications 2 à 8, **caractérisée en que** l'élément de blocage (54) est conçu comme une broche (56) s'étendant axialement et sollicitée par ressort.

10. Soupape à touches selon la revendication 8 ou 9, **caractérisée en ce que** le cran (44) est conçu comme un élément (46) à élasticité radiale.

11. Soupape à touches selon la revendication 9 ou 10, **caractérisée en ce que** la broche (56) présente sur l'extrémité attribuée à l'élément (46) élastique un chanfrein (64), qui est en prise avec l'élément (46) à élasticité radiale lors du desserrage du cran (44).

12. Soupape à touches selon l'une quelconque des revendications 9 à 11, **caractérisée en ce que** la broche (56) est réalisée comme une broche (56) disposée sur le côté extérieur (66) du corps de soupape.

13. Soupape à touches selon l'une quelconque des revendications 8 à 12, **caractérisée en ce que** l'élément élastique (46) est réalisé comme un ressort barre (46) prétendu radialement vers l'intérieur.

14. Soupape à touches selon la revendication 13, **caractérisée en ce que** dans le poussoir de soupape (28) est évidée une rainure (52), dans laquelle le ressort barre (46) prétendu radialement peut être encliqueté en engagement bloquant.

15. Soupape à touches selon l'une quelconque des revendications 1 à 14, **caractérisée en ce que** le poussoir de soupape (28) est réceptionné dans un vissage de soupape (22), qui présente au moins deux cavités de doigt (23).

16. Soupape à touches selon la revendication 15, **caractérisée en ce qu'**un contour d'encoche des cavités de doigt (23) est conçu de telle sorte qu'un flanc de cavité (27) est fortement marqué dans le sens du desserrage.

17. Soupape à touches selon la revendication 16, **caractérisée en ce que** le flanc de cavité (27) augmente dans le sens du dévissage dans la direction radiale plus fortement que le flanc de cavité (29) opposé dans le sens du vissage.

18. Soupape à touches selon la revendication 17, **caractérisée en ce que** le flanc de cavité (29) fait place en direction du dévissage à un arrondi (31) périphérique.

19. Instrument médical pour l'aspiration et de lavage, **caractérisé en ce qu'**il présente une soupape à touches (20) montée selon l'une quelconque des revendications 1 à 18.
